# EUROPEAN PATENT SPECIFICATION

(11) **EP 3 531 922 B1**
(45) Date of publication and mention of the grant of the patent: **29.04.2020**
(21) Application number: 17784985.8
(22) Date of filing: 24.10.2017
(51) Int. Cl.: A61B 8/12, A61B 8/14, A61B 8/00, A61B 8/08

(54) **INTERVENTIONAL INSTRUMENT COMPRISING AN ULTRASOUND TRANSDUCER**
INTERVENTIONELLES INSTRUMENT MIT EINEM ULTRASCHALLWANDLER
INSTRUMENT D'INTERVENTION COMPRENANT UN TRANSDUCTEUR ULTRASONORE

(30) Priority: 26.10.2016 EP 16195779
(43) Date of publication of application: 04.09.2019
(73) Proprietor: Koninklijke Philips N.V., 5656 AG Eindhoven (NL)
(72) Inventor: HAKKENS, Franciscus Johannes Gerardus, 5656 AE Eindhoven (NL); HENDRIKS, Cornelis Petrus, 5656 AE Eindhoven (NL); VAN DER LINDE, Franciscus Reinier Antonius, 5656 AE Eindhoven (NL); LUCASSEN, Gerhardus Wilhelmus, 5656 AE Eindhoven (NL); HOVENKAMP, Ronald Antonie, 5656 AE Eindhoven (NL); VAN DER HORST, Arjen, 5656 AE Eindhoven (NL); GIJSBERS, Gerardus Henricus Maria, 5656 AE Eindhoven (NL); PELSSERS, Eduard Gerard Marie, 5656 AE Eindhoven (NL); HARKS, Godefridus Antonius, 5656 AE Eindhoven (NL)
(74) Representative: Fairley, Peter Douglas
(86) International application number: PCT/EP2017/077211
(87) International publication number: WO 2018/077909

(56) References cited:
- EP-A1- 1 829 483
- WO-A1-2005/011504
- WO-A2-2009/140641
- WO-A2-2012/054926
- US-A1- 2004 056 751
- US-A1- 2007 066 901
- US-A1- 2007 167 821
- US-A1- 2009 216 125
- US-B2- 8 652 050

## Description

### FIELD OF THE INVENTION

The invention relates to an interventional instrument comprising an ultrasound transducer, an ultrasound system comprising the interventional instrument with the ultrasound transducer, and a control method for controlling the ultrasound system. The invention relates further to a computer program for controlling the ultrasound system.

### BACKGROUND OF THE INVENTION

WO 2012/054926 A2 a catheter comprising an elongate catheter body, a distal end portion supportably disposed at a distal end of the catheter body and defining an enclosed volume containing a fluid, and an ultrasound transducer immersed within the fluid and disposed for oscillating, pivotal movement through an angular range about a pivot axis within the enclosed volume, wherein the pivot axis is fixed relative to the distal end portion. The catheter further comprises first and second shape memory members operatively associated with the ultrasound transducer, wherein the first shape memory member can be actuated by causing a change in state of the first shape memory member, wherein the second shape memory member can be actuated by causing a change in state of the second shape memory member and wherein the first and second shape memory members are actuatable in at least partially-offset timed relation to affect at least a portion of the oscillating, pivotal movement of the ultrasound transducer.

US 2007/0167821 A1 discloses a rotating transducer array assembly for use in volumetric ultrasound imaging procedures. The assembly comprises a transducer array, a motion controller coupled to the transducer array for rotating the transducer array, and an interconnect assembly coupled to the transducer array for transmitting signals between the transducer and an imaging device, wherein the interconnection assembly is configured to reduce its respective torque load on the transducer and motion controller due to a rotating motion of the transducer.

US 2009/0216125 A1 discloses an imaging device for emitting ultrasonic acoustic waves and providing a useable image in response to detection of reflections of the acoustic waves. The imaging device comprises an axially elongate structure adapted for insertion into a body lumen or cavity, an array of outwardly directed transmitting transducer elements mounted to the structure for electrically generating a plurality of output ultrasonic acoustic waves, and a first linear actuator operably connected to the structure for rotationally vibrating the array of transducer elements circumferentially around the longitudinal axis of the axially elongate structure, wherein the first linear actuator is affixed to the structure proximate the distal end of the structure. The imaging device further comprises a second linear actuator operably connected to the structure for rotationally vibrating the array of transducer elements around an offset axis which is offset from the longitudinal axis of the axially elongate structure, wherein the second linear actuator is affixed to the structure proximate the distal end of the structure, and a cable connecting the structure to an environment external of the lumen and including a signal channel for transporting electrical signals. Moreover, the imaging device comprises a receiving transducer mounted on the structure and proximate to the array of transmitting transducer elements for receiving reflections of the output ultrasonic acoustic waves from the array of transducer elements and converting the reflection ultrasonic acoustic waves to reflection electrical signals that are transmitted along the signal channel in the cable, and a processor responsive to the reflection electrical signals from the cable for providing imaging data from the reflection electrical signals. Finally, the imaging device comprises a display responsive to the imaging data for providing a visual image of the body lumen or cavity and its surrounding structure, and an element disposed at the distal tip of the axially elongate structure, which is adapted for performing an endovascular medical intervention.

US 8,652,050 B2 discloses an intravascular imaging device comprising an elongate member having a proximal portion and a distal portion, and an actuator mechanism disposed adjacent to the distal portion of the elongate member. The actuator mechanism comprises a first anchor, a second anchor, a movable element, a deformable component and a two-way shape memory alloy actuator, wherein an imaging element is connected to the movable element. The first anchor is connected to a first side of the movable element by the two-way shape memory alloy actuator and the second anchor is connected to a second side of the movable element by the deformable component, wherein the first and second anchors are secured relative to the elongate member and wherein a longitudinal axis of the actuator mechanism extends between the first anchor and the second anchor parallel to the longitudinal axis of the elongate member. The movable element and the imaging element are moved relative to the elongate member upon activation of the two-way shape memory alloy actuator. This technique for moving the movable element and the imaging element needs relatively much space.

### SUMMARY OF THE INVENTION

It is an object of the present invention to provide an interventional instrument comprising an ultrasound transducer which can be more compact and require less space. It is a further object of the present invention to provide an ultrasound system comprising the interventional instrument, and a control method and a computer program for controlling the ultrasound system.

In a first aspect of the present invention an interventional instrument comprising an ultrasound unit and a rotation unit for rotating the ultrasound unit is presented, wherein the rotation unit comprises an actuation element being:
an electroactive polymer for actuating the rotation of the ultrasound unit, wherein the ultrasound unit is arranged within a housing of the interventional instrument, wherein the rotation unit comprises a support means on which the ultrasound unit is rotatably supported, wherein the support means and the housing form a first space and a second space, wherein the rotation unit comprises a pump with the electroactive polymer, wherein the pump is adapted to pump a filling material from the first space to the second space, wherein the housing, the support means and the ultrasound unit are arranged such that the ultrasound unit is rotated, if the filling material is pumped from the first space to the second space, or
a one-way shape memory alloy for actuating the rotation of the ultrasound unit, wherein the one-way shape memory alloy is formed as a coil which is configured to provide a rotating force for rotating the ultrasound unit from an initial position to a rotated position, wherein the rotation unit comprises a force providing element for providing a force for forcing the ultrasound unit back from the rotated position to the initial position.

If a two-way shape memory alloy is used for actuating the rotation, the two-way shape memory alloy needs to be relatively thick and the mechanism for moving the ultrasound transducer needs to be relatively large, in order to provide a force being sufficient for moving the ultrasound unit. However, if a rotation unit comprises, as an actuation element for actuating a rotation of an ultrasound unit, an electroactive polymer or a one-way shape memory alloy, the rotation unit with the actuation element can be more compact and smaller.

The rotation unit comprises an actuation element being an electroactive polymer or a one-way shape memory alloy. This means the rotation unit can comprise a) one or more electroactive polymer actuation elements and no one-way shape memory alloy actuation element, b) one or more one-way shape memory alloy actuation elements and no electroactive polymer actuation element or c) one or more electroactive polymer actuation elements and one or more one-way shape memory alloy actuation elements. Thus, for instance, in an embodiment, besides an actuation element being one of the electroactive polymer and the one-way shape memory alloy, the rotation unit can comprise a further actuation element being the other of the electroactive polymer and the one-way shape memory alloy.

The interventional instrument is preferentially an elongated interventional instrument like a catheter, a guidewire, a needle, an endoscope, et cetera. The rotation unit is preferentially adapted to rotate the ultrasound unit around a longitudinal axis of the interventional instrument. The electroactive polymer is preferentially a dielectric electroactive polymer. However, the electroactive polymer can also be an ionic polymer.

The coil is preferentially configured to provide a rotating force by itself for rotating the ultrasound unit from an initial position to a rotated position. Thus, it is preferentially not necessarily required to use a further one-way shape memory alloy for generating the rotating force.

In an embodiment, especially in an embodiment in which the actuation element is the one-way shape memory alloy, the ultrasound unit is arranged within a housing at a tip of the interventional instrument, wherein the rotation unit is adapted to rotate the ultrasound unit relative to the housing. Thus, the ultrasound transducer can be rotated within the housing, without moving the housing. In particular, the outside of the interventional instrument can remain unchanged, i.e., for instance, the interventional instrument itself can remain at a fixed position, while the ultrasound transducer is rotated. The positioning of the interventional instrument can therefore be decoupled from the rotation of the ultrasound transducer such that, for example, the rotation of the ultrasound transducer does not influence the position of the interventional instrument. In another embodiment, especially in an embodiment in which the actuation element is the one-way shape memory alloy, the ultrasound unit is arranged at a tip of the interventional instrument such that the ultrasound unit is moved, if the tip of the interventional instrument is moved, wherein the rotation unit is adapted to rotate the tip, in order to rotate the ultrasound unit.

In an embodiment the rotation unit comprises a support means on which the ultrasound unit is rotatably supported. In particular, the support means forms a rotational axis around which the ultrasound unit is rotatable, wherein the rotational axis is parallel to a longitudinal axis of the interventional instrument. For example, the actuation element can be attached to the ultrasound unit and can be adapted to actuate the rotation of the ultrasound unit by changing at least one of its dimension and shape. This allows for a further reduced size of the rotation unit and hence of the interventional instrument. In this embodiment the actuation element is preferentially the electroactive polymer. It should be noted that "at least one of A and B" is to be understood as including a) A without B, b) B without A, and c) A and B.

In an embodiment the ultrasound unit is arranged within a housing at a tip of the interventional instrument, wherein the support means and the housing form a first space and a second space, wherein the rotation unit comprises a pump with the electroactive polymer, wherein the pump is adapted to pump a filling material from the first space to the second space, wherein the housing, the support means and the ultrasound unit are arranged such that the ultrasound unit is rotated, if the filling material is pumped from the first space to the second space. The filing material is preferentially a fluid or a gel. Also this embodiment allows for a further reduced size of the interventional instrument.

In a preferred embodiment the interventional instrument is adapted to receive the filling material from an external filling material source, i.e. initially the interventional instrument itself may not comprise the filing material. For instance, only when being in use the filing material may be filled from the external filing material source into the first space, into the second space, or into the first space and the second space. In this case the filing material is preferentially saline. However, the filing material can also be another fluid or a gel. The housing can comprise an opening for allowing the filing material to leave the interventional instrument at its tip. However, the housing can also not comprise such an opening, in order to not allow the filing material to leave the interventional instrument at its tip.

In another preferred embodiment the first space, the second space, or the first space and the second space already comprise the filling material. Thus, in this embodiment the filing material is already present within the interventional instrument. In particular, at a manufacturing site the first space, the second space, or the first space and the second space, can already be filled with the filing material.

In an embodiment the one-way shape memory alloy is formed as a coil which is configured to provide a rotating force for rotating the ultrasound unit from an initial position to a rotated position. In particular, the one-way shape memory alloy is a coiled wire. The coil, which is preferentially a coiled wire, can be integrated with a housing at a tip of the interventional instrument or with another part of the interventional instrument like a shaft, which is rigidly attached to the housing, wherein the ultrasound unit can be arranged within the housing such that the ultrasound unit is rotated with the housing, if the coil is actuated. The coil is preferentially arranged such that its axis, i.e. preferentially its longitudinal axis, is parallel to the longitudinal axis of the interventional instrument, wherein this includes that the axis of the coil is on the longitudinal axis of the interventional instrument or has a non-zero distance to the longitudinal axis. Moreover, the rotation unit can comprise a force providing element for providing force for forcing the ultrasound unit back from the rotated position to the initial position. The force providing element can be a spring or another element forcing the ultrasound unit back from the rotated position to the initial position. If the force providing element is a spring, preferentially it is also arranged such that its axis is parallel to the longitudinal axis of the interventional instrument. Also the force providing element can be integrated with a housing at a tip of the interventional instrument or with another part of the interventional instrument like a shaft, which is rigidly attached to the housing. Also this embodiment allows for an interventional instrument having relatively small dimensions, wherein nevertheless enough force can be generated for rotating the ultrasound transducer.

In a further aspect of the present invention an ultrasound system is presented, wherein the ultrasound system comprises a) an interventional instrument comprising an ultrasound unit and a rotation unit as defined in claim 1, wherein the ultrasound unit is adapted to generate ultrasound signals, and b) a controlling unit for controlling the rotation unit by controlling the actuation element, in order to control a rotational position of the ultrasound unit. Preferentially, the ultrasound imaging system further comprises c) an ultrasound image generation unit for generating several ultrasound images for different rotational positions of the ultrasound unit based on ultrasound signals received from the ultrasound unit, d) a display for displaying the generated several ultrasound images, and e) a user interface for allowing a user to select an ultrasound image among the displayed several ultrasound images, wherein the controlling unit is adapted to control the rotation unit such that the ultrasound unit is rotated to the rotational position which corresponds to the selected ultrasound image. Thus, a user can select a desired rotational position of the ultrasound transducer in a relatively simple way, wherein the user just needs to select an ultrasound image showing, for instance, an interesting feature.

In a further aspect of the present invention a control method for controlling the ultrasound system as defined in claim 11 is presented, wherein the control method comprises controlling the rotation unit of the interventional instrument of the ultrasound system by controlling the actuation element being
an electroactive polymer for actuating the rotation of the ultrasound unit, wherein the ultrasound unit is arranged within a housing of the interventional instrument, wherein the rotation unit comprises a support means on which the ultrasound unit is rotatably supported, wherein the support means and the housing form a first space and a second space, wherein the rotation unit comprises a pump with the electroactive polymer, wherein the pump is adapted to pump a filling material from the first space to the second space, wherein the housing, the support means and the ultrasound unit are arranged such that the ultrasound unit is rotated, if the filling material is pumped from the first space to the second space, or
a one-way shape memory alloy for actuating the rotation of the ultrasound unit, wherein the one-way shape memory alloy is formed as a coil which is configured to provide a rotating force for rotating the ultrasound unit from an initial position to a rotated position, wherein the rotation unit comprises a force providing element for providing a force for forcing the ultrasound unit back from the rotated position to the initial position,
in order to control a rotational position of the ultrasound unit of the interventional instrument.

In a further aspect of the present invention a computer program for controlling the ultrasound system as defined in claim 11 is presented, wherein the computer program comprises program code means for causing the ultrasound system to carry out the controlling method as defined in claim 13, when the computer program is run on the ultrasound system.

It shall be understood that the interventional instrument of claim 1, the ultrasound system of claim 11, the control method of claim 13, and the computer program of claim 14 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of an ultrasound system,
Figs. 2 to 4 show schematically and exemplarily an embodiment of a tip of an interventional instrument of the ultrasound system,
Figs. 5 and 6 show schematically and exemplarily a further embodiment of a tip of the interventional instrument of the ultrasound system,
Fig. 7 show schematically and exemplarily a further embodiment of a tip of the interventional instrument of the ultrasound system,
Figs. 8 and 9 show schematically and exemplarily a further embodiment of a tip of the interventional instrument of the ultrasound system, and
Fig. 10 shows a flowchart exemplarily illustrating an embodiment of a control method for controlling the ultrasound system.

### DETAILED DESCRIPTION OF EMBODIMENTS

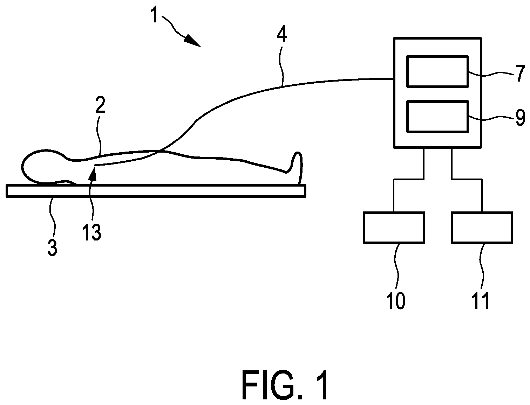

Fig. 1 shows schematically and exemplarily an embodiment of an ultrasound system 1. The ultrasound system 1 comprises an interventional instrument 4 being, in this embodiment, a catheter. The tip 13 of the interventional instrument 4 has been inserted into a patient 2 lying on a patient table 3. In particular, the tip 13 of the interventional instrument 4 has been inserted into the heart of the patient 2.

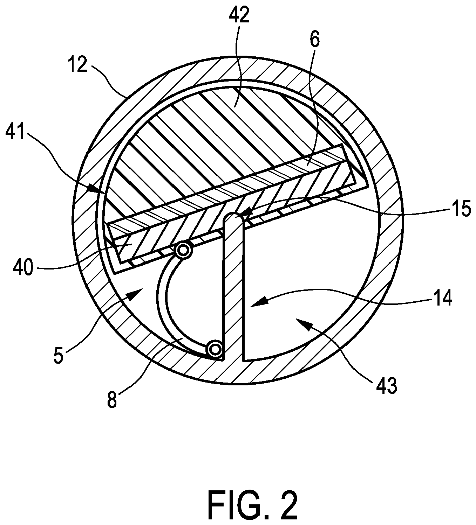

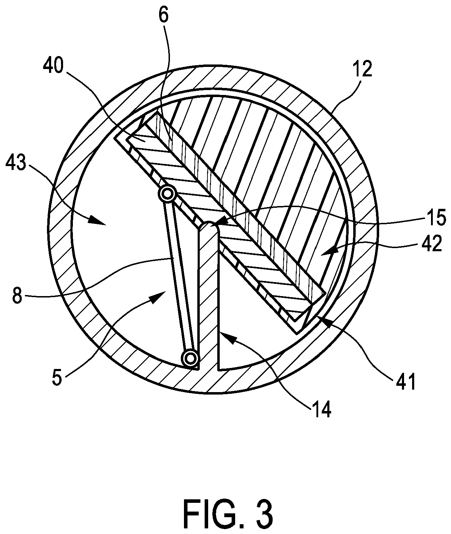

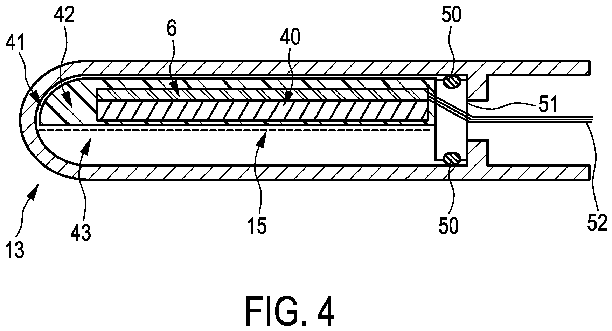

Figs. 2 and 3 schematically and exemplarily show a cross section and Fig. 4 shows a longitudinal section of the tip 13 of the interventional instrument 4. As illustrated in these figures, the tip 13 of the interventional instrument 4 comprises an ultrasound unit 41 with a transducer 6 on a backing 40. The ultrasound unit 41 is adapted to send ultrasound waves into the surrounding and to receive ultrasound waves from the surrounding.

The ultrasound unit 41 is rotatably supported on a support means 14. The support means 14 forms a rotational axis 15 of the tip 13 of the interventional instrument 4, wherein the rotational axis 15 is parallel to the longitudinal axis of the tip 13 of the interventional instrument 4. The ultrasound unit 41 is rotatable around this rotational axis 15.

The tip 13 of the interventional instrument 4 further comprises a housing 12 having a circular cross section. Moreover, the ultrasound unit 41 comprises an acoustic window 42 on top of an ultrasound waves emitting and receiving surface of the ultrasound transducer 6. The acoustic window 42 is made of an acoustically transparent material which, in this embodiment, is not only arranged on top of the ultrasound waves emitting and receiving surface of the ultrasound transducer 6, but which encloses the ultrasound transducer 6 and the backing 40. The outer shape of the acoustic window 42 corresponds to the circular cross section of the housing 12, in order to allow for a rotation of the ultrasound unit 41 relative to the housing 12. In particular, the acoustic window 42 is shaped like a cylinder section. The acoustic window 42 is preferentially made of a polymer.

In this embodiment the support means 14 and the housing 12 are integrally formed as a single element. A first end of an actuation element 8 is attached to the ultrasound unit 41 and an opposing second end of the actuation element 8 is attached to the support means 14 or the housing 12. In this embodiment the second end of the actuation element 8 is attached to a region where the support means 14 transitions into the housing 12, i.e. the second end of the actuation element 8 is attached to the base of the support means 14 or to the housing 12 close to the base of the support means 14.

The actuation element 8 is an electroactive polymer which changes its shape from bending to straightening and vice versa in response to an electric field, in order to actuate the rotation of the ultrasound unit 41 relative to the housing 12. For example, in Fig. 2 the actuation element 8 is bent such that the ultrasound unit 41 has been rotated to the left side and in Fig. 3 the actuation element 8 has been straightened such that the ultrasound unit 41 has been rotated to the right side. The housing 12 is filled with a gel 43.

Since the actuation element 8 and the support means 14, on which the ultrasound unit 41 is rotatably supported, are adapted to rotate the ultrasound unit 41, these components can be regarded as being components of a rotation unit 5 for rotating the ultrasound unit 41.

The housing, which can also be regarded as being an outer jacket of the tip 13 of the interventional instrument 4, is an extruded profile, wherein the support means 14 is also a part of the extruded profile. The gel 43, with which the housing 12 is filled, is preferentially a low viscosity gel. The ultrasound transducer 6 can be configured to allow for an electronic steering of the ultrasound waves, i.e. of an ultrasound beam. Thus, on top of the physical rotation of the ultrasound transducer 6, which already leads to an enlarged field of view, the field of view can be further enlarged by the electronic steering.

The rotation unit 5 with the actuation element 8 is integrated in the distal end of the interventional instrument 4, i.e. in the tip 13 of the interventional instrument 4, such that a rotation of the ultrasound unit 41 is not resulting in a net force on the tip 13, thereby keeping an ultrasound imaging plane stable and maintaining an axial orientation.

As can be seen in Fig. 4, the space within the housing 12 at the tip 13 of the interventional instrument 4 is sealed from the rest of the interventional instrument 4 by using a plug 51 containing electrical feedthroughs allowing for an electrical connection of the ultrasound transducer 6 and the actuation element 8 from the outside of the interventional instrument 4. In Fig. 4 for clarity reasons only the electrical connections 52 for electrically connecting the ultrasound transducer 6 are shown. However, of course also electrical connections for connecting the actuation element 8 are present. The sealing of the inner space at the tip 13 of the interventional instrument 4 from the rest of the interventional instrument 4 is further improved by using a sealing ring 50.

The ultrasound system 1 further comprises a controlling unit 7 for controlling the rotation unit 5 by controlling the actuation element 8, in order to control the rotational position of the ultrasound unit 41. In particular, the control unit 7 is electrically connected to the actuation element 8, in order to control the actuation element 8 and hence the rotational position of the ultrasound unit 41 by applying a corresponding electric field. Moreover, the ultrasound system 1 comprises an ultrasound image generation unit 9 for generating an ultrasound image based on ultrasound signals received from the ultrasound unit 41. In particular, the ultrasound image generation unit 9 is electrically connected to the ultrasound transducer 6, in order to control the ultrasound transducer 6 and in order to receive ultrasound signals from the ultrasound transducer 6. The ultrasound system 1 also comprises a user interface 10 for allowing a user to provide inputs into the ultrasound system 1, and a display 11 for displaying a generated ultrasound image. In particular, the ultrasound image generation unit 9 is adapted to generate several ultrasound images for different rotational positions of the ultrasound unit 41 based on the ultrasound signals received from the ultrasound unit 41 and the display 11 is adapted to display the generated several ultrasound images. Furthermore, the user interface 10 can be adapted to allow the user to select an ultrasound image and hence a corresponding rotational position of the ultrasound unit 41 in which the ultrasound unit 41 was arranged while acquiring the ultrasound signal used for generating this selected ultrasound image. The controlling unit 7 can then be adapted to control the rotation unit 5 such that the ultrasound unit 41 is rotated to this rotational position which corresponds to the selected ultrasound image.

Thus, the ultrasound system 1 can be adapted to enable an automatic rotation to a selected ultrasound image. The tip of the interventional instrument can record a set of ultrasound images while the ultrasound unit is rotated, wherein these images can be displayed on the display 11 for manual selection of a preferred ultrasound image and hence, for instance, of a preferred ultrasound image plane. After this selection, the ultrasound unit can be rotated to the corresponding rotational position, i.e. to the corresponding rotation angle, in order to generate and display, for instance, a B-mode ultrasound image or an M-mode ultrasound image of the selected slice, i.e. of the selected ultrasound imaging plane.

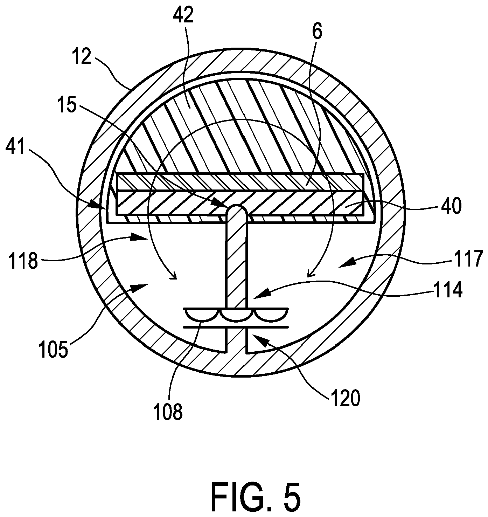

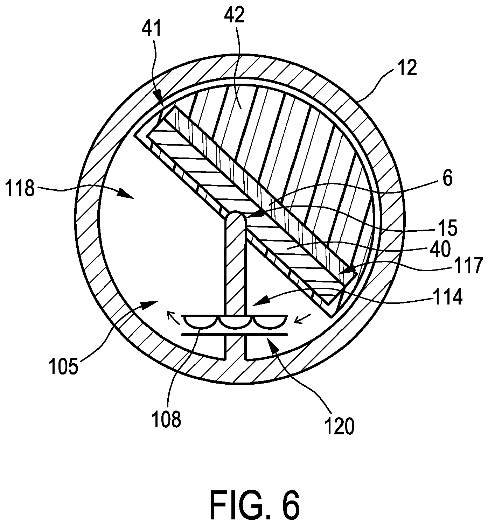

Figs. 5 and 6 schematically and exemplarily illustrate a further embodiment of the tip of the interventional instrument 4. Also in this embodiment the tip of the interventional instrument 4 comprises an ultrasound unit 41 with an ultrasound transducer 6, a backing 40 and an acoustic window 42. Moreover, also in this embodiment a support means 114 rotatably supports the ultrasound unit 41 on a rotational axis 15 being parallel to the longitudinal axis of the tip of the interventional instrument 4, in order to allow for a rotation of the ultrasound unit 41 relative to the housing 12.

The support means 114 and the housing 12 form a first space 117 and a second space 118, wherein these two spaces 117, 118 are separated from each other by the support means 114. Both spaces 117, 118 are filled with a filling material and the filling material can be transferred from the first space 117 to the second space 118 and vice versa via a pump 120 integrated in the support means 114. The pump 120 comprises an electroactive polymer 108 and is adapted to a) pump the filling material from the first space 117 to the second space 118, b) from the second space 118 to the first space 117, or c) not pump the filling material, depending on an actuation state of the electroactive polymer 108, wherein the actuation state is controllable by the controlling unit 7 by applying a corresponding electric field.

By pumping the filling material from one of the two spaces 117, 118 to the other of the two spaces 117, 118 the ultrasound unit 41 can be rotated around the rotational axis 15. For instance, in Fig. 6 the filling material has been pumped from the first space 117 to the second space 118, thereby rotating the ultrasound unit 41 to the right.

The electroactive polymer pump 120 is quite flexible, can easily be shaped in a desired form factor and is silent. The pump 120 can be, for instance, a peristaltic pump which might use several valves in a row. In Figs. 5 and 6 three of these valves are schematically and exemplarily illustrated. The pump 120 moves the filling material from one side of the support means 114, which might also be regarded as being a support bar, to the other side. In this way a very large deflection can be realized, wherein also in this embodiment the rotation can be combined with an electronic steering of the ultrasound beam. This can allow for a field of view of, for instance, 360 degrees. The filling material can be a gel or a fluid.

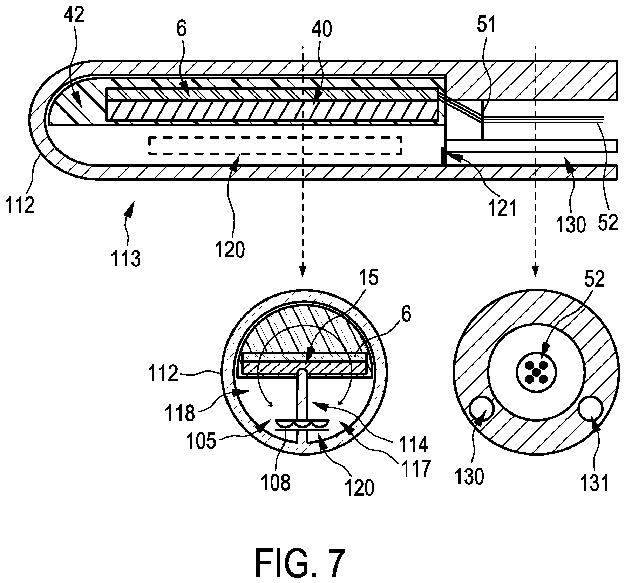

In the embodiment described above with reference to Figs. 5 and 6 the filling material is preferentially located within the first and second spaces 117, 118, not only if the interventional instrument 4 is used, but also before and after this use. In particular, the filling material might be permanently within these spaces 117, 118 and might only be exchanged for maintenance purposes. In another embodiment the filling material may be introduced into the first and second spaces 117, 118, only if the interventional instrument is used. In this case the interventional instrument 4 is adapted to receive the filling material from an external filling material source which might comprise a pump for pumping the filling material into the first and second spaces 117, 118. Thus, the interventional instrument can generally be completely dry without a gel or a fluid in the housing at the tip of the interventional instrument, wherein only in use this housing is flushed with the filling material which in this case is preferentially saline. The filling material provides acoustic coupling and allows for the rotation of the ultrasound unit via the above described pumping mechanism. Fig. 7 schematically and exemplarily illustrates a tip of an interventional instrument which is assembled dry and which is flushed with saline during the interventional procedure.

In Fig. 7 a longitudinal section and two cross sections of the tip of the interventional instrument are illustrated. At least one of the first space 117 and the second space 118 is fluidly connected with the external filling material source through two channels 130, 131, wherein one of these channels is used for introducing the filling material from the outside of the interventional instrument into the first and second spaces 117, 118 and wherein a second channel 131 is adapted for allowing the introduced filling material to leave the first and second spaces 117, 118. A closing element 121 can be located within at least one of the first and second channels 130, 131, in order to, for instance, close the first and second spaces 117, 118 after these spaces have been filled with the filling material. The closing element 121 is preferentially a valve which might also be actuated by an electroactive polymer which can be electrically connected to the controlling unit 7 for controlling the valve from the outside of the interventional instrument.

Although in Fig. 7 the distal tip of the housing 112 is closed such that the filling material cannot leave the housing 112 at its distal tip, in another embodiment the housing 112 can also comprise an opening at its distal tip, wherein this opening may be equipped with a valve, in order to control when which amount of the filling material should leave the casing 112 at the distal tip. Thus, not only the inflow of the filling material into the housing 112, in particular into the first and second spaces 117, 118, can be controlled, but also an outflow out of the housing 112 through a distal opening at the distal tip of the casing 112 can be controlled by using a valve. Also this valve for controlling the outflow of the filling material through the distal opening at the distal tip of the housing 112 can be actuated by using an electroactive polymer which can be electrically connected with the controlling unit 7 for allowing the controlling unit 7 to also control this valve.

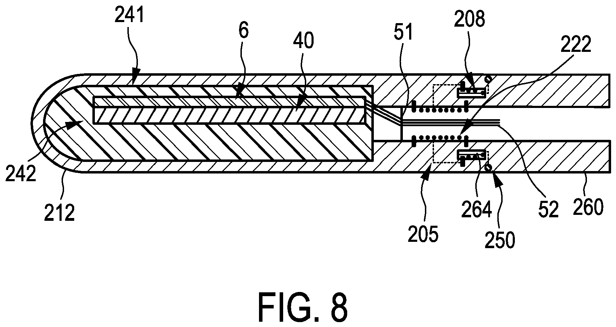

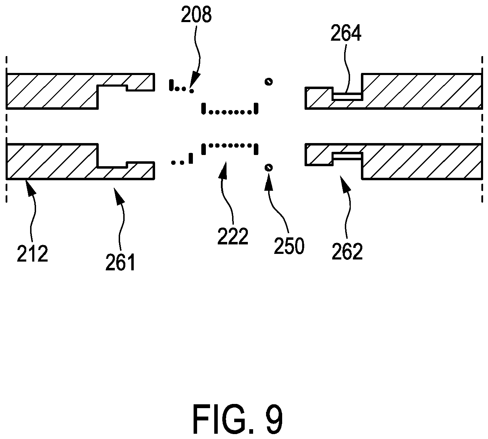

Figs. 8 and 9 schematically and exemplarily illustrate a further embodiment of the tip of the interventional instrument 4. In this embodiment an ultrasound transducer 6 is arranged on a backing 40, wherein the ultrasound transducer 6 and the backing 40 are enclosed within an acoustically transparent material 242 which is preferentially an acoustically transparent polymer. The acoustically transparent material 242 is transparent for ultrasound waves transmitted by the ultrasound transducer 6 and received by the ultrasound transducer 6. The ultrasound transducer 6, the backing 40 and the ultrasonically transparent material 242 can be regarded as being components of an ultrasound unit 241.

The ultrasound unit 241 is arranged within and enclosed by a housing 212 of the tip of the interventional instrument such that the ultrasound unit 241 is moved, if the tip of the interventional instrument 4 and hence the housing 212 is moved. In this embodiment the interventional instrument further comprises a coiled wire 208 made of a one-way shape memory alloy. In comparison to a two-way shape memory alloy, using a one-way shape memory alloy allows for a smaller and more compact rotation unit. Moreover, the one-way shape memory alloy has a more stable performance over time in comparison to a two-way shape memory alloy.

The coiled wire 208 is configured to provide a rotating force for rotating the housing 212 of the tip of the interventional instrument and thereby the ultrasound unit 241 from an initial position to a rotated position. Moreover, the interventional instrument comprises an antagonist coil 222, which can also be regarded as being a spring, for forcing the ultrasound unit 241 back from the rotated position to the initial position. The coiled wire 208 made of the one-way shape memory alloy is coiled around a ring 264 which is preferentially stiff, i.e. which has preferentially a stiffness such that it does not change its shape, if the coils 208, 222 are used for rotating the ultrasound unit 241. The coiled wire 208 can provide a relatively large stroke for rotating the ultrasound unit 241. The antagonist coil 222 is used to reset the coiled wire 208 to the detwinned martensite state after cooling from the austenite state and to bring the ultrasound unit 241 back into its original orientation, i.e. into its initial position.

Fig. 9 illustrates some components of the embodiment shown in Fig. 8 separated from each other. As can be seen in this figure, the housing 212 of the tip of the interventional instrument comprises a first interlocking element 261, and a shaft 260 of the interventional instrument comprises corresponding second interlocking elements 262 with the stiff ring 264. The first and second interlocking elements 261, 262 are adapted such that the housing 212 of the tip can be engaged with the shaft 260 of the interventional instrument by using a mechanical lock.

A sealing ring 250 is used for sealing the engaging region from the outside, in order to make this connection between the housing 212 of the tip and the shaft 260 fluid tight. The antagonist coil 222 is preferentially configured such that it provides a continuous compressive force on the sealing ring 250. The coiled wire 208 is electrically connected with the controlling unit 7, in order to allow the controlling unit 7 to actuate the coiled wire 8 by electrical heating. The corresponding electrical connections are not shown in Fig. 8 for clarity reasons.

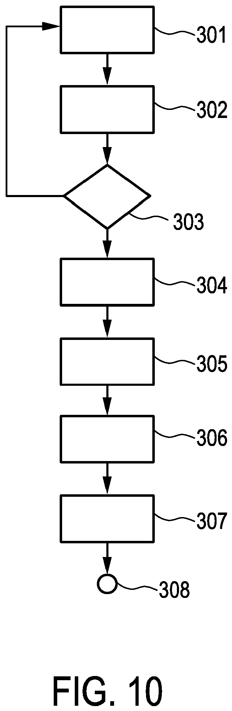

In the following an embodiment of a control method for controlling the ultrasound system will exemplarily be described with reference to a flowchart shown in Fig. 10.

After the tip of the interventional instrument has been introduced into the patient 2, in particular, into the heart of the patient 2, in step 301 the ultrasound unit at the tip of the interventional instrument 4 is rotated to a first rotational position by controlling the actuation element of the rotation unit via the controlling unit 7. In step 302 an ultrasound image of the interior of the patient 2 is generated at the current rotational position of the ultrasound unit. In step 303 it is determined whether the ultrasound unit should be rotated to a further rotational position, in order to generate a further ultrasound image at this further rotational position. If this is the case, the method proceeds with step 301. Otherwise, the method proceeds with step 304. For instance, in step 303 it can be checked whether an abort criterion for generating ultrasound images at different rotational positions is fulfilled. This abort criterion can relate to, for instance, a predefined number of rotational positions at which ultrasound images should be generated, a predefined angular range over which ultrasound images should be generated, et cetera. The abort criterion can also be whether a user has indicated via the user interface 10 that the generation of the ultrasound images should be stopped.

In step 304 the generated ultrasound images are shown on the display 11 and in step 305 the user is allowed to select an ultrasound image among the displayed several ultrasound images by using the user interface 10. In step 306 the rotation unit is controlled such that the ultrasound unit is rotated to the rotational position which corresponds to the selected ultrasound image and in step 307 one or several further ultrasound images are generated at the rotational position to which the ultrasound unit has been rotated in step 306. The control method ends in step 308.

Electroactive polymers can easily be manufactured into various shapes allowing for an easy integration into a rotation unit for rotating an ultrasound unit. They require a relatively low power, have a relatively small form factor, are very flexible, noiseless, can be accurately manufactured, provide a fast response and can be reversibly used. The electroactive polymers can be field driven or ionic driven. For instance, as a field driven electroactive polymer an electrostrictive polymer like a dielectric elastomer, a polyvinylidene fluoride (PVDF) based relaxor polymer, a liquid crystal elastomer (LCE), et cetera can be used. As an ionic driven electroactive polymer a conjugated polymer, a carbon nanotube (CNT) - polymer composite, an ionic polymer metal composite (IPMC), et cetera can be used. For actuating a field driven electroactive polymer preferentially an electric field is used through direct electromechanical coupling, while the actuation mechanism for an ionic electroactive polymer involves the diffusion of ions.

It is known to rotate an ultrasound transducer in an interventional instrument like a catheter based on a mechanical force transfer from a proximal end of the interventional instrument to a distal end of the interventional instrument. As a result of this, the tip of the interventional instrument will move by the force that is applied over a shaft of the interventional instrument, wherein this shaft cannot be straight, but has to follow a non-straight path through the arteries. These interventional instruments are rather stiff and complex. Contrary to this, the embodiments described above with reference to Figs. 1 to 9 allow for a rotation of an ultrasound unit, which can also include a flexing of a tip of the interventional instrument comprising the ultrasound unit, without requiring a mechanical force transfer from the proximal end to the distal end of the interventional instrument. The distal tip of the interventional instrument can therefore remain relatively stable, i.e. it can remain substantially at the same stable position, while generating ultrasound images in different directions. This can also simplify a generation of a three-dimensional ultrasound image based on the several ultrasound images which can be two-dimensional and which have been generated at known spatial positions. Moreover, since ultrasound images can be generated in different directions, while the position of the distal tip of the interventional instrument can remain substantially stable, a user like a physician can look around within the patient without losing orientation. A mechanical force transfer from the proximal end to the distal end would cause the distal end to move in an uncontrolled manner so that the orientation would be lost. Moreover, known ultrasound transducers have a limited field of view for imaging, even if the ultrasound transducer is an array having an increased field of view due to beam forming. By rotating the ultrasound transducer, i.e. by rotating the ultrasound unit, the field of view can be further increased. Preferentially, beam forming, i.e. electrical steering of the ultrasound beam, is combined with the rotation of the ultrasound unit for increasing the field of view. A field of view of 360° can be possible.

Although in above described embodiments the interventional instrument is preferentially a catheter, the interventional instrument can also be another kind of interventional instrument like a guidewire, a needle, an endoscope, et cetera. Moreover, although in above described embodiments certain electroactive polymers and one-way shape memory alloys have been described, which initiate the rotation of the ultrasound unit in different ways, also other electroactive polymers and one-way shape memory alloys, respectively, can be used which may initiate the rotation of the ultrasound unit in another way. The rotation of the ultrasound unit can be caused, for instance, by a bending, stretching, swelling, shrinking, et cetera of the electroactive polymer or one-way shape memory alloy. The electroactive polymer or one-way shape memory alloy can be adapted to indirectly cause the rotation of the ultrasound unit by initiating an intermediate action which leads to the rotation like the above described pumping of a filling material from a first space to a second space which leads to the rotation of the ultrasound unit.

Although in above described embodiments the ultrasound unit is used for imaging purposes, the ultrasound unit can also be used for a non-imaging ultrasound sensing. The interventional instrument with the ultrasound unit can be an intracardiac echocardiography (ICE) catheter.

The embodiments described above with reference to Figs. 1 to 9 each comprise a single actuation mechanism for rotating the ultrasound unit by using an electroactive polymer or a one-way shape memory alloy. It is also possible that at least two of these actuation mechanisms are combined in a single embodiment. For instance, the use of the electroactive polymer 8 described above with reference to Figs. 2 and 3 can be combined with the pump 108 described above with reference to Figs. 5 to 7. Although specific actuation mechanisms have been described above with reference to Figs. 1 to 9, also other actuation mechanisms can be used for rotating the ultrasound unit, which use at least one of an electroactive polymer and a one-way shape memory alloy.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Operations like the control of the rotation unit for rotating the ultrasound unit, the generation of the ultrasound images et cetera performed by one or several units or devices can be performed by any other number of units or devices. These operations and/or the control of the ultrasound system in accordance with the control method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to an interventional instrument like a catheter comprising an ultrasound unit and a rotation unit for rotating the ultrasound unit, wherein the rotation unit comprises an actuation element being an electroactive polymer or a one-way shape memory alloy for actuating the rotation of the ultrasound unit. Since the rotation unit comprises, as an actuation element for actuating the rotation of the ultrasound unit, an electroactive polymer or a one-way shape memory alloy, the rotation unit with the actuation element can be very compact and small.

## Claims

1. An interventional instrument comprising an ultrasound unit (41) and a rotation unit (5; 105; 205) for rotating the ultrasound unit (41; 241), wherein the rotation unit (5; 105; 205) comprises an actuation element (8; 108; 208) being:
an electroactive polymer (8; 108) for actuating the rotation of the ultrasound unit (41), wherein the ultrasound unit is arranged within a housing (12; 112) of the interventional instrument (4), wherein the rotation unit (5; 105) comprises a support means (114) on which the ultrasound unit (41) is rotatably supported, wherein the support means (114) and the housing (12; 112) form a first space (117) and a second space (118), wherein the rotation unit (105) comprises a pump (120) with the electroactive polymer (108), wherein the pump (120) is adapted to pump a filling material from the first space (117) to the second space (118), wherein the housing (112), the support means (114) and the ultrasound unit (41) are arranged such that the ultrasound unit (41) is rotated, if the filling material is pumped from the first space (117) to the second space (118), or
a one-way shape memory alloy for actuating the rotation of the ultrasound unit (41), wherein the one-way shape memory alloy (208) is formed as a coil which is configured to provide a rotating force for rotating the ultrasound unit (241) from an initial position to a rotated position, wherein the rotation unit (205) comprises a force providing element (222) for providing a force for forcing the ultrasound unit (241) back from the rotated position to the initial position.

2. The interventional instrument as defined in claim 1, wherein the ultrasound unit (41) is arranged within a housing (12; 112) at a tip (13; 113) of the interventional instrument (4), wherein the rotation unit (5; 105; 205) is adapted to rotate the ultrasound unit (41) relative to the housing (12; 112; 212).

3. The interventional instrument as defined in claim 1, wherein the ultrasound unit (241) is arranged at a tip of the interventional instrument (4) such that the ultrasound unit (241) is moved, if the tip of the interventional instrument (4) is moved, wherein the rotation unit (205) is adapted to rotate the tip, in order to rotate the ultrasound unit (241).

4. The interventional instrument as defined in claim 3, wherein the coil is integrated with the housing or with another part of the interventional instrument (4), which is rigidly attached to the housing.

5. The interventional instrument as defined in claim 1, wherein the support means (14; 114) forms a rotational axis (15) around which the ultrasound unit (41) is rotatable, wherein the rotational axis (15) is parallel to a longitudinal axis of the interventional instrument (4).

6. The interventional instrument as defined in claim 5, wherein the actuation element (8) is attached to the ultrasound unit (41) and is adapted to actuate the rotation of the ultrasound unit (41) by changing at least one of its dimension and shape.

7. The interventional instrument as defined in claim 1, wherein the interventional instrument (4) is adapted to receive the filling material from an external filling material source.

8. The interventional instrument as defined in claim 1, wherein the first space (117), the second space (118), or the first space (117) and the second space (118) comprise the filling material.

9. The interventional instrument as defined in claim 1, wherein the interventional instrument (4) has a longitudinal axis, wherein the axis of the coil, which is formed by the one-way shape memory alloy, is parallel to the longitudinal axis of the interventional instrument.

10. The interventional instrument as defined in claim 1, wherein the force providing element is a spring.

11. An ultrasound system comprising:
- an interventional instrument (4) comprising an ultrasound unit (41; 241) and a rotation unit (5; 105; 205) as defined in claim 1, wherein the ultrasound unit (41; 241) is adapted to generate ultrasound signals, and
- a controlling unit (7) for controlling the rotation unit (5; 105; 205) by controlling the actuation element (8; 108; 208), in order to control a rotational position of the ultrasound unit (41).

12. The ultrasound system as defined in claim 11, wherein the ultrasound imaging system further comprises:
- an ultrasound image generation unit (9) for generating several ultrasound images for different rotational positions of the ultrasound unit (41; 241) based on ultrasound signals received from the ultrasound unit (41; 241),
- a display (11) for displaying the generated several ultrasound images, and
- a user interface (10) for allowing a user to select an ultrasound image among the displayed several ultrasound images,
wherein the controlling unit (7) is adapted to control the rotation unit (5; 105; 205) such that the ultrasound unit (41; 241) is rotated to the rotational position which corresponds to the selected ultrasound image.

13. A control method for controlling the ultrasound system as defined in claim 11, wherein the control method comprises controlling the rotation unit (5; 105; 205) of the interventional instrument (4) of the ultrasound system by controlling the actuation element (8; 108; 208) being
an electroactive polymer for actuating the rotation of the ultrasound unit (41), wherein the ultrasound unit is arranged within a housing (12; 112) of the interventional instrument (4), wherein the rotation unit (5; 105) comprises a support means (114) on which the ultrasound unit (41) is rotatably supported, wherein the support means (114) and the housing (12; 112) form a first space (117) and a second space (118), wherein the rotation unit (105) comprises a pump (120) with the electroactive polymer (108), wherein the pump (120) is adapted to pump a filling material from the first space (117) to the second space (118), wherein the housing (112), the support means (114) and the ultrasound unit (41) are arranged such that the ultrasound unit (41) is rotated, if the filling material is pumped from the first space (117) to the second space (118), or
a one-way shape memory alloy for actuating the rotation of the ultrasound unit (41), wherein the one-way shape memory alloy (208) is formed as a coil which is configured to provide a rotating force for rotating the ultrasound unit (241) from an initial position to a rotated position, wherein the rotation unit (205) comprises a force providing element (222) for providing a force for forcing the ultrasound unit (241) back from the rotated position to the initial position,
in order to control a rotational position of the ultrasound unit (41; 241) of the interventional instrument (4).

14. A computer program for controlling the ultrasound system as defined in claim 11, the computer program comprising program code means for causing the ultrasound system to carry out the controlling method as defined in claim 13, when the computer program is run on the ultrasound system.

## Patentansprüche

1. Ein Interventionsinstrument, das aus einem Ultraschallgerät (41) und einer Rotationseinheit (5; 105; 205) zum Drehen des Ultraschallgeräts (41; 241) besteht, wobei die Rotationseinheit (5; 105; 205) ein Betätigungselement (8; 108; 208) umfasst, bei dem es sich um Folgendes handelt:
ein elektroaktives Polymer (8; 108) zum Betätigen der Drehung des Ultraschallgeräts (41), wobei das Ultraschallgerät in einem Gehäuse (12; 112) des Interventionsinstruments (4) angebracht ist. Hierbei besteht die Rotationseinheit (5; 105) aus einer Führungsvorrichtung (114), auf der das Ultraschallgerät (41) drehend geführt wird, wobei die Führungsvorrichtung (114) und das Gehäuse (12; 112) einen ersten (117) und einen zweiten Raum (118) bilden. Hierbei besteht die Rotationseinheit (105) zudem aus einer Pumpe (120) mit dem elektroaktiven Polymer (108), wobei die Pumpe (120) ein Füllmaterial aus dem ersten (117) in den zweiten Raum (118) pumpt, und wobei das Gehäuse (112), die Führungsvorrichtung (114) und das Ultraschallgerät (41) so angeordnet sind, dass das Ultraschallgerät (41) gedreht wird, wenn das Füllmaterial vom ersten (117) in den zweiten Raum (118) gepumpt wird, oder
einer Einweg-Formgedächtnislegierung zum Betätigen der Drehung des Ultraschallgeräts (41), wobei die Einweg-Formgedächtnislegierung (208) aus einer Spule gebildet wird, die die Drehkraft zum Drehen des Ultraschallgeräts (241) aus einer ursprünglichen Position in eine gedrehte Position bereitstellt. Hierbei besteht die Rotationseinheit (205) aus einem Kraft erzeugenden Element (222), das die Kraft bereitstellt, um das Ultraschallgerät (241) aus der gedrehten Position zurück in die ursprüngliche Position zu drehen.

2. Das Interventionsinstrument gemäß Anspruch 1, wobei das Ultraschallgerät (41) in einem Gehäuse (12; 112) an der Spitze (13; 113) des Interventionsinstruments (4), angebracht ist und die Rotationseinheit (5; 105; 205) das Ultraschallgerät (41) relativ zum Gehäuse (12; 112; 212) dreht.

3. Das Interventionsinstrument gemäß Anspruch 1, wobei das Ultraschallgerät (241) so an der Spitze des Interventionsinstruments (4) angebracht ist, dass das Ultraschallgerät (241) bewegt wird, wenn die Spitze des Interventionsinstruments (4) bewegt wird. Hierbei dreht die Rotationseinheit (205) die Spitze, um das Ultraschallgerät (241) zu drehen.

4. Das Interventionsinstrument gemäß Anspruch 3, wobei die Spule in das Gehäuse oder in ein anderes, starr am Gehäuse angebrachten Teil des Interventionsinstruments (4) integriert ist.

5. Das Interventionsinstrument gemäß Anspruch 1, wobei die Führungsvorrichtung (14; 114) eine Drehachse (15) bildet, um die das Ultraschallgerät (41) gedreht werden kann. Hierbei verläuft die Drehachse (15) parallel zur Längsachse des Interventionsinstruments (4).

6. Das Interventionsinstrument gemäß Anspruch 5, wobei das Betätigungselement (8) am Ultraschallgerät (41) angebracht ist und die Drehung des Ultraschallgeräts (41) durch mindestens eine Veränderung der Abmessung oder Form betätigt.

7. Das Interventionsinstrument gemäß Anspruch 1, wobei das Interventionsinstrument (4) das Füllmaterial aus einer externen Füllmaterialquelle erhält.

8. Das Interventionsinstrument gemäß Anspruch 1, wobei sich das Füllmaterial im ersten (117), im zweiten (118) oder im ersten (117) und im zweiten Raum (118) befindet.

9. Das Interventionsinstrument gemäß Anspruch 1, wobei das Interventionsinstrument (4) über eine Längsachse verfügt, wobei die durch die Einweg-Formgedächtnislegierung gebildete Achse der Spule parallel zur Längsachse des Interventionsinstruments verläuft.

10. Das Interventionsinstrument gemäß Anspruch 1, wobei es sich beim Kraft erzeugenden Element um eine Feder handelt.

11. Ein Ultraschallsystem, das aus Folgendem besteht:
- einem Interventionsinstrument (4), das aus einem Ultraschallgerät (41; 241) und einer Rotationseinheit (5; 105; 205) gemäß Anspruch 1 besteht, wobei das Ultraschallgerät (41; 241) Ultraschallsignale erzeugt, und
- einer Steuerungseinheit (7) zum Steuern der Rotationseinheit (5; 105; 205), indem das Betätigungselement (8; 108; 208) geregelt wird, um die Drehposition des Ultraschallgeräts (41) zu steuern.

12. Das Ultraschallsystem gemäß Anspruch 11, wobei das Ultraschall-Bildgebungssystem zudem Folgendes umfasst:
- ein Ultraschall-Bildgebungsgerät (9) zum Generieren mehrerer Ultraschallbilder aus verschiedenen Drehpositionen des Ultraschallgeräts (41; 241) beruhend auf den Ultraschallgerät (41; 241) erhaltenen Ultraschallsignalen,
- eine Anzeige (11) zum Anzeigen der generierten Ultraschallbilder, und
- eine Benutzeroberfläche (10), über die der Benutzer eines der Ultraschallbilder für die Anzeige auswählen kann,
wobei die Steuerungseinheit (7) die Rotationseinheit (5; 105; 205) so steuert, dass das Ultraschallgerät (41; 241) in die Drehposition gedreht wird, die dem ausgewählten des Ultraschallbild entspricht.

13. Eine Steuerungsmethode zum Steuern des Ultraschallsystems gemäß Anspruch 11, wobei die Steuerungsmethode das Steuern der Rotationseinheit (5; 105; 205) des Interventionsinstruments (4) des Ultraschallsystems durch Regeln des Betätigungselements (8; 108; 208) umfasst, bei dem es sich um Folgendes handelt:
ein elektroaktives Polymer zum Betätigen der Drehung des Ultraschallgeräts (41), wobei das Ultraschallgerät in einem Gehäuse (12; 112) des Interventionsinstruments (4) angebracht ist. Hierbei besteht die Rotationseinheit (5; 105) aus einer Führungsvorrichtung (114), auf der das Ultraschallgerät (41) drehend geführt wird, wobei die Führungsvorrichtung (114) und das Gehäuse (12; 112) einen ersten (117) und einen zweiten Raum (118) bilden. Hierbei besteht die Rotationseinheit (105) zudem aus einer Pumpe (120) mit dem elektroaktiven Polymer (108), wobei die Pumpe (120) ein Füllmaterial aus dem ersten (117) in den zweiten Raum (118) pumpt, und wobei das Gehäuse (112), die Führungsvorrichtung (114) und das Ultraschallgerät (41) so angeordnet sind, dass das Ultraschallgerät (41) gedreht wird, wenn das Füllmaterial vom ersten (117) in den zweiten Raum (118) gepumpt wird, oder
einer Einweg-Formgedächtnislegierung zum Betätigen der Drehung des Ultraschallgeräts (41), wobei die Einweg-Formgedächtnislegierung (208) aus einer Spule gebildet wird, die die Drehkraft zum Drehen des Ultraschallgeräts (241) aus einer ursprünglichen Position in eine gedrehte Position bereitstellt. Hierbei besteht die Rotationseinheit (205) aus einem Kraft erzeugenden Element (222), das die Kraft bereitstellt, um das Ultraschallgerät (241) aus der gedrehten Position zurück in die ursprüngliche Position zu drehen,
um die Drehposition des Ultraschallgeräts (41; 241) des Interventionsinstruments (4) zu steuern.

14. Ein Computerprogramm zum Steuern des Ultraschallsystems gemäß Anspruch 11, wobei das Computerprogramm Programmcode umfasst, der das Ultraschallsystem dazu veranlasst, die Steuerungsmethode gemäß Anspruch 13 auszuführen, wenn das Computerprogramm im Ultraschallsystem ausgeführt wird.

## Revendications

1. Instrument interventionnel comprenant une unité à ultrasons (41) et une unité de rotation (5; 105; 205) pour faire tourner l'unité à ultrasons (41; 241), dans lequel l'unité de rotation (5; 105; 205) comprend un élément d'actionnement (8; 108; 208) étant:
un polymère électroactif (8; 108) pour actionner la rotation de l'unité à ultrasons (41),
dans lequel l'unité à ultrasons est disposée à l'intérieur d'un boîtier (12; 112) de l'instrument interventionnel (4),
dans lequel l'unité de rotation (5; 105) comprend un moyen de support (114) sur lequel l'unité à ultrasons (41) est supportée en rotation,
dans lequel le moyen de support (114) et le boîtier (12; 112) forment un premier espace (117) et un second espace (118), dans lequel l'unité de rotation (105) comprend une pompe (120) comportant le polymère électroactif (108), dans lequel la pompe (120) est conçue pour pomper une matière de remplissage du premier espace (117) vers le second espace (118), dans lequel le boîtier (112), le moyen de support (114) et l'unité à ultrasons (41) sont disposés de telle sorte que l'unité à ultrasons (41) tourne, lorsque la matière de remplissage est pompée du premier espace (117) vers le second espace (118); ou
un alliage à mémoire de forme unidirectionnelle pour actionner la rotation de l'unité à ultrasons (41), dans lequel l'alliage à mémoire de forme unidirectionnelle (208) est formé sous la forme d'une bobine, laquelle est conçue pour fournir une force de rotation pour faire tourner l'unité à ultrasons (241) d'une position initiale à une position tournée, dans lequel l'unité de rotation (205) comprend un élément de fourniture de force (222) pour fournir une force pour forcer l'unité à ultrasons (241) de la position tournée à la position initiale.

2. Instrument interventionnel selon la revendication 1, dans lequel l'unité à ultrasons (41) est disposée à l'intérieur d'un boîtier (12; 112) au niveau d'une pointe (13; 113) de l'instrument interventionnel (4), dans lequel l'unité de rotation (5; 105; 205) est conçue pour faire tourner l'unité à ultrasons (41) par rapport au boîtier (12; 112; 212).

3. Instrument interventionnel selon la revendication 1, dans lequel l'unité à ultrasons (241) est disposée à une pointe de l'instrument interventionnel (4) de telle sorte que l'unité à ultrasons (241) est déplacée lorsque la pointe de l'instrument interventionnel (4) est déplacée, dans lequel l'unité de rotation (205) est conçue pour faire tourner la pointe pour faire tourner l'unité à ultrasons (241).

4. Instrument interventionnel selon la revendication 3, dans lequel la bobine est intégrée au boîtier ou à une autre partie de l'instrument interventionnel (4), laquelle est fixée rigidement au boîtier.

5. Instrument interventionnel selon la revendication 1, dans lequel le moyen de support (14; 114) forme un axe de rotation (15) autour duquel l'unité à ultrasons (41) peut tourner, dans lequel l'axe de rotation (15) est parallèle à un axe longitudinal de l'instrument interventionnel (4).

6. Instrument interventionnel selon la revendication 5, dans lequel l'élément d'actionnement (8) est fixé à l'unité à ultrasons (41) et ledit élément d'actionnement est conçu pour actionner la rotation de l'unité à ultrasons (41) en modifiant au moins une parmi sa dimension et sa forme.

7. Instrument interventionnel selon la revendication 1, dans lequel l'instrument interventionnel (4) est conçu pour recevoir la matière de remplissage d'une source de matière de remplissage externe.

8. Instrument interventionnel selon la revendication 1, dans lequel le premier espace (117), le second espace (118) ou le premier espace (117) et le second espace (118) comprennent la matière de remplissage.

9. Instrument interventionnel selon la revendication 1, dans lequel l'instrument interventionnel (4) comporte un axe longitudinal, dans lequel l'axe de la bobine, lequel est formé par l'alliage à mémoire de forme unidirectionnelle, est parallèle à l'axe longitudinal de l'instrument interventionnel.

10. Instrument interventionnel selon la revendication 1, dans lequel l'élément de fourniture de force est un ressort.

11. Système à ultrasons comprenant:
- un instrument interventionnel (4) comprenant une unité à ultrasons (41; 241) et une unité de rotation (5; 105; 205) selon la revendication 1, dans lequel l'unité à ultrasons (41; 241) est conçue pour générer des signaux ultrasonores; et
- une unité de commande (7) pour commander l'unité de rotation (5; 105; 205) en commandant l'élément d'actionnement (8; 108; 208) pour commander une position de rotation de l'unité à ultrasons (41).

12. Système à ultrasons selon la revendication 11, dans lequel le système d'imagerie à ultrasons comprend en outre:
- une unité de génération des images ultrasonores (9) pour générer une pluralité d'images ultrasonores pour différentes positions de rotation de l'unité ultrasonore (41; 241) en fonction des signaux ultrasonores reçus de l'unité ultrasonore (41; 241);
- un affichage (11) pour afficher la pluralité d'images ultrasonores générées; et
- une interface utilisateur (10) pour permettre à un utilisateur de sélectionner une image ultrasonore parmi la pluralité d'images ultrasonores affichées, dans lequel l'unité de commande (7) est conçue pour commander l'unité de rotation (5; 105; 205) de telle sorte que l'unité à ultrasons (41; 241) est tournée vers la position de rotation, laquelle correspond à l'image ultrasonore sélectionnée.

13. Procédé de commande permettant de commander le système à ultrasons tel que défini dans la revendication 11,
dans lequel le procédé de commande comprend la commande de l'unité de rotation (5; 105; 205) de l'instrument interventionnel (4) du système à ultrasons en commandant l'élément d'actionnement (8; 108; 208) étant
un polymère électroactif pour actionner la rotation de l'unité à ultrasons (41), dans lequel l'unité à ultrasons est disposée à l'intérieur d'un boîtier (12; 112) de l'instrument interventionnel (4), dans lequel l'unité de rotation (5; 105) comprend un moyen de support (114) sur lequel l'unité à ultrasons (41) est supportée en rotation, dans lequel le moyen de support (114) et le boîtier (12; 112) forment un premier espace (117) et un second espace (118), dans lequel l'unité de rotation (105) comprend une pompe (120) comportant le polymère électroactif (108), dans lequel la pompe (120) est conçue pour pomper une matière de remplissage du premier espace (117) vers le second espace (118),
dans lequel le boîtier (112), le moyen de support (114) et l'unité à ultrasons (41) sont disposés de telle sorte que l'unité à ultrasons (41) tourne lorsque la matière de remplissage est pompée du premier espace (117) vers le second espace (118); ou
un alliage à mémoire de forme unidirectionnelle pour actionner la rotation de l'unité à ultrasons (41), dans lequel l'alliage à mémoire de forme unidirectionnelle (208) est formé en tant qu'une bobine, laquelle est conçue pour fournir une force de rotation pour faire tourner l'unité à ultrasons (241) d'une position initiale à une position tournée,
dans lequel l'unité de rotation (205) comprend un élément de fourniture de force (222) pour fournir une force pour forcer l'unité à ultrasons (241) de la position tournée à la position initiale pour commander une position de rotation de l'unité à ultrasons (41; 241) de l'instrument interventionnel (4).

14. Programme informatique permettant de commander le système à ultrasons selon la revendication 11, ledit programme informatique comprenant des moyens de code programme pour amener le système à ultrasons à mettre en œuvre le procédé de commande, tel que défini dans la revendication 13, lorsque le programme informatique est exécuté sur le système à ultrasons.
